# DEMANDE DE BREVET EUROPEEN

(11) **EP 2 158 942 A2**
(43) Date de publication de la demande: **03.03.2010**
(21) Numéro de dépôt: 08169494.5
(22) Date de dépôt: 24.08.2006
(51) Int. Cl.: A61Q 5/06, A61K 8/04, A61K 8/37, A61K 8/81, A61K 8/87, A61K 8/88, A61K 8/85, A61K 8/41, A61K 8/34

(54) **Composition de coloration directe comprenant des composés oxygénés insolubles, procédés mettant en oeuvre cette composition**

(30) Priorité: 25.08.2005 FR 0508755
(62) Demande divisionnaire de: 06291349.6
(71) Demandeur: L'Oréal, 75008 Paris (FR)
(72) Inventeur: Kravtchenko, Sylvain 168 Ji Nan Lu The Lakeville, 200021, Shangai (CN); Dubief, Claude, 78150, Le Chesnay (FR)
(74) Mandataire: Dossmann, Gérard

(57) **Abrégé**

L'invention porte sur une composition de coloration directe aqueuse non-oxydante des matières kératiniques, telles que les fibres kératiniques humaines, en particulier les cheveux, comprenant dans un milieu cosmétiquement acceptable :
(a) au moins un colorant direct hydrosoluble,
(b) au moins 30% en poids d'au moins un composé organique oxygéné non colorant insoluble dans l'eau, par rapport au poids total de ladite composition,

ainsi que sur un procédé de coloration mettant en oeuvre ladite composition.

## Description

La présente invention a trait à des compositions de coloration directe aqueuse non-oxydante des matières kératiniques, telles que fibres kératiniques humaines et en particulier des cheveux comprenant un composé insoluble dans l'eau et un procédé de coloration directe des fibres kératiniques mettant en oeuvre ladite composition.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho-ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs, tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser pauser pour permettre aux molécules colorées de pénétrer, par diffusion, à l'intérieur du cheveu, puis à rincer les fibres.

Contrairement aux compositions de teinture par oxydation, les compositions de teinture directes ou semi-permanentes sont mises en oeuvre sans la présence obligatoire d'un agent oxydant. Ces teintures peuvent être effectuées de manière répétée sans dégrader la fibre kératinique.

Il est connu par exemple d'utiliser des colorants directs nitrés benzèniques, anthraquinoniques, nitropyridiniques, azoïques, xanthéniques, acridiniques, aziniques ou triarylméthaniques.

Le temps de pause d'une composition de coloration directe est classiquement compris entre 15 et 45 minutes selon la nature de la fibre (sensibilisée ou non) et la nature du colorant utilisée. Depuis longtemps, des recherches sont menées pour diminuer le temps de pause des compositions colorantes, sans pour autant augmenter la concentration des constituants et tout en conservant un bon niveau de coloration, c'est-à-dire une bonne puissance tinctoriale, une bonne tenue de la couleur face aux agents extérieurs et dans le temps.

Il existe un réel besoin de disposer de compositions de coloration directe améliorées en termes d'efficacité, de rapidité de réaction ou de pénétration du colorant dans la fibre, tout en conservant une bonne innocuité, une bonne ténacité et une bonne sélectivité, cette dernière résultant de la différence de montée entre différentes parties d'un cheveu ou d'une chevelure.

De manière surprenante et avantageuse, la Demanderesse vient de découvrir que l'utilisation de compositions pour la teinture des fibres kératiniques, en particulier des fibres kératiniques humaines, telles que les cheveux comprenant un colorant direct particulier et un composé particulier insoluble dans l'eau en une certaine quantité, permet ces améliorations.

Outre leur avantage en terme d'innocuité, ces compositions permettent de diminuer le temps de pause de la composition sur les fibres à colorer et permettent l'obtention de reflets homogènes et tenaces.

En outre, ces compositions présentent un bon profil toxicologique.

L'invention concerne également un procédé de coloration direct mettant en oeuvre cette composition.

D'autres caractéristiques, aspects, objets et avantages de l'invention figurant dans la description ci-dessous permettront de mieux cerner l'invention.

La présente invention a donc pour objet une composition de coloration directe aqueuse non-oxydante des matières kératiniques, telles que fibres kératiniques humaines et en particulier des cheveux comprenant dans un milieu cosmétiquement acceptable :
(a) au moins un colorant direct hydrosoluble,
(b) au moins 30% en poids d'au moins un composé organique oxygéné non colorant particulier et insoluble dans l'eau par rapport au poids total de ladite composition.

Par composé organique oxygéné, on entend au sens de la présente invention, tout composé organique comportant au moins un atome d'oxygène dans sa structure moléculaire élémentaire.

Par composé organique oxygéné non colorant non soluble, on entend au sens de la présente invention, tout composé organique oxygéné inapte à colorer les cheveux à partir des compositions de l'invention et présentant une solubilité dans l'eau à température ambiante (25°C) inférieure à 0,5 % en poids.

Par composition non oxydante, on entend au sens de la présente invention toute composition ne contenant pas de composé organique peroxygéné tel que le peroxyde d'hydrogène, les persulfates, les perbonates, les peracides.

La composition tinctoriale conforme à l'invention comprend un ou plusieurs colorants directs hydrosolubles classiquement utilisés en coloration directe et de toute polarité.

Par colorant hydrosoluble, on entend au sens de la présente invention, un colorant dont la solubilité à 25°C et à pH7 dans l'eau et/ou dans un mélange eau (85 % en poids) et hexylène glycol (15 % en poids) est supérieure ou égale à 0,1 %.

Les colorants directs utilisés selon l'invention sont choisis parmi les colorants nitrés de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO 95/01772, EP 714954, FR 2 822 696, FR 2 825 702, FR 2 825 625, FR 2 822 698, FR 2 822 693, FR 2 822 694, FR 2 829 926, FR 2 807 652, WO 02/078660, WO 02/100834, WO 02/100369 et FR 2 844 269 dont le contenu fait partie intégrante de l'invention.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Acid Violet 43
- Acid Blue 62
- Basic Blue 22
- Basic Blue 99
   ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer le composé suivant :
- 2-β-hydroxyéthlyamino-5-[bis-(β-4'-hydroxyéthyl)amino]anilino-1,4-benzoquinone

Parmi les colorants directs naturels utilisables selon l'invention, on peut citer l'acide carminique, l'acide kermésique, la purpurogalline, le protocatéchaldéhyde, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20 % en poids environ du poids total de la composition de coloration, et encore plus préférentiellement de 0,005 à 10 % en poids environ.

Les composés organiques oxygénés insolubles selon l'invention sont choisis parmi les polyamides 6, 66, 11, les polyesters, les polyuréthanes, les polycyanoacrylates, les polyméthacrylates de méthyle, les polycarbonates, le téflon, les résines ou élastomères de silicone.

On peut citer les alcools gras comportent de 8 à 40 atomes de carbone, les esters et éthers gras d'acides ou d'alcools gras, les chaînes grasses de ces acides gras ou alcools gras comportent de 8 à 40 atomes de carbone et étant éventuellement hydroxylées. A titre d'exemple citons : le mono et le di-stéarate d'éthylène glycol, le monooléate de pentaerythritol, le tristéarate de sorbitan, le dioléate de glycérol, les esters, amides et éthers gras d'éthylène glycol, de propylène glycol, le distéaryléther, l'alcool stéarylique, l'alcool béhénylique, l'alcool cétyl stéarylique.

La teneur en composé organique oxygéné insoluble dans l'eau dans la composition de coloration est supérieure à 30% en poids de la composition de coloration, de préférence est comprise entre 30 et 90 % en poids de la composition de coloration, et plus particulièrement est comprise entre 30 et 60 % en poids de la composition de coloration.

Ainsi lorsque que le ou les composés insolubles dans l'eau selon l'invention sont solides, la composition est sous forme d'une suspension. Lorsque le ou les composés insolubles dans l'eau selon l'invention sont sous forme liquide, par exemple sous forme d'une phase organique insoluble dans la phase aqueuse, la composition est une émulsion, voire une dispersion.

Le milieu approprié pour la teinture appelé aussi support de teinture est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le glycérol, les monoéthers de polyols, tels que le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

La composition selon l'invention contient de 0,1 à 69,995 % d'eau, de préférence 10 à 68 %, plus préférentiellement de 20 à 65 % et encore plus préférentiellement de 30 à 60 % en poids par rapport au poids total de la composition tinctoriale.

Les solvants organiques sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs et des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12, de préférence entre 5 et 11, et encore plus particulièrement de 6 à 8,5. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines, telles que les mono-, di- et triéthanolamines ainsi que leurs dérivés, les hydroxydes de sodium ou de potassium et les composés de formule (II) suivante : 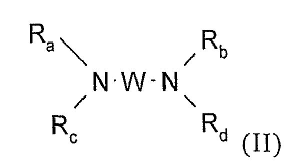 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition selon l'invention peut résulter du mélange extemporanée de 2 compositions ou plus. Par exemple, la composition peut être obtenue à partie du mélange d'un support de coloration comprenant le ou les composés organiques oxygénés insolubles dans l'eau et d'une composition aqueuse comprenant le colorant direct.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains. Il en est de même pour les compositions, qui suite à leur mélange conduisent à la composition selon l'invention, sous réserve que l'une d'entre elles soit une solution aqueuse.

Le procédé de la présente invention est un procédé dans lequel on applique sur les fibres la composition selon la présente invention telle que définie précédemment, puis on rince lesdites fibres.

De préférence, la composition tinctoriale selon l'invention est appliquée sur les fibres kératiniques, puis est laissée poser pendant une période comprise entre 3 minutes et 1 heure, et de préférence entre 15 minutes et 45 minutes, lesdites fibres sont ensuite rincée.

L'invention porte également sur l'utilisation de la composition tinctoriale selon l'invention pour la teinture des fibres kératiniques.

Les exemples suivants servent à illustrer l'invention sans toutefois présenter un caractère limitatif.

| | |
|---|---|
| Distéaryléther | 34g |
| Laurylsulfate de sodium | 2g |
| Brij 700 | 1g |
| Ammonium acryloydimethyl laurate/steareth 20 methacrylate crosspolymer | 2g |
| Colorant direct hydrosoluble | 0,15g |
| 2-amino-2-méthyl-1-propanol | qs pH 8,5 |
| Eau déminéralisée | qsp 100g |

| | | |
|---|---|---|
| Example 1: | colorant direct hydrosoluble | Basic Red 51 |
| Example 2: | colorant direct hydrosoluble | Acid Red 35 |
| Example 3: | colorant direct hydrosoluble | Acide Carminique |
| Example 4: | 1-β-hydroxyéthylamino-2-nitro-4-amino-benzène | |

Les compositions des 4 exemples sont appliquées 30 minutes à température ambiante sur des cheveux 90% blancs. Après rinçage et séchage, les cheveux sont teints avec une coloration auburn plus marquée, que si le distéaryléther était remplacé par un composé non colorant soluble dans l'eau tel que l'éthanol.

Des résultats similaires peuvent être obtenus en remplaçant le distéaryléther poids pour poids par de la poudre Nylon-6 proposée par Induchem sous la dénomination Inducos.

## Revendications

1. Composition de coloration directe aqueuse non-oxydante des matières kératiniques, telles que les fibres kératiniques humaines, en particulier les cheveux, comprenant dans un milieu cosmétiquement acceptable :
(a) au moins un colorant direct hydrosoluble, dont la solubilité à 25°C et à pH 7 dans l'eau et/ou dans un mélange eau (85 % en poids) et hexylène glycol (15 % en poids) est supérieure ou égale à 0,1 %,
(b) au moins 30% en poids d'au moins un composé organique oxygéné non colorant insoluble dans l'eau, c'est-à-dire présentant une solubilité dans l'eau à température ambiante (25°C) inférieure à 0,5 % en poids, par rapport au poids total de ladite composition choisi parmi les polyamides 6, 66, 11, les polyesters, les polyuréthanes, les polycyanoacrylates, les polyméthacrylates de méthyle, les polycarbonates, le téflon, les résines ou élastomères de silicone.

2. Composition selon la revendication 1, **caractérisée en ce que** le colorant direct est choisi parmi les colorants nitrés de la série benzénique neutres, acides ou cationiques, les colorants directs azoïques neutres, acides ou cationiques, les colorants directs quinoniques, anthraquinoniques neutres, acides ou cationiques, les colorants directs aziniques, les colorants directs triarylméthaniques, les colorants directs indoaminiques et les colorants directs naturels.

3. Composition selon la revendication 2, **caractérisée en ce que** le colorant direct est choisi parmi :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(p-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène,
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium,
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- 1-(4'-aminodiphénylazo)-2-méthyl-4bis-(β-hydroxyéthyl) aminobenzène
- l'acide 4-hydroxy-3-(2-méthoxyphénylazo)-1-naphtalène sulfonique.
- Acid Violet 43
- Acid Blue 62
- Basic Blue 22
- Basic Blue 99
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.
- Basic Blue 17
- Basic Red 2.
- Basic Green 1
- Acid blue 9
- Basic Violet 3
- Basic Violet 14
- Basic Blue 7
- Acid Violet 49
- Basic Blue 26
- Acid Blue 7
- 2-β-hydroxyéthlyamino-5-[bis-(β-4'-hydroxyéthyl)amino]anilino-1,4-benzoquinone
ainsi que l'acide carminique, l'acide kermésique, la purpurogalline, le protocatéchaldéhyde, l'isatine, la curcumine, la spinulosine, l'apigénidine.

4. Composition selon l'une quelconque des revendications précédentes, **caractérisée en ce que** le ou les colorants directs hydrosolubles représentent de préférence de 0,001 à 20 % en poids environ du poids total de la composition, et encore plus préférentiellement de 0,005 à 10 % en poids environ.

5. Composition selon l'une quelconque des revendications précédentes, **caractérisée en ce que** la teneur en composé organique oxygéné insoluble dans l'eau et/ou dans la composition de coloration est comprise entre 30 et 90 % en poids de la composition de coloration, et plus particulièrement est comprise entre 30 et 60 % en poids de la composition de coloration.

6. Composition selon l'une quelconque des revendications précédentes, **caractérisée en ce qu'**elle contient de 0,1 à 69,995 % d'eau, de préférence 10 à 68 %, plus préférentiellement 20 à 65 % et en particulier 30 à 60 % en poids par rapport au poids total de la composition.

7. Composition selon l'une quelconque des revendications précédentes, telle qu'elle comprend au moins un solvant hydroxylé tel que l'éthanol, le propylène glycol, le glycérol, les mono éthers de polyols.

8. Composition selon l'une quelconque des revendications précédentes, telle qu'elle comprend au moins un adjuvant choisi parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, et en particulier les épaississants associatifs polymères anioniques, cationiques, non ioniques et amphotères, les agents antioxydants, les agents de pénétration, les agents séquestrants, les parfums, les tampons, les agents dispersants, les agents conditionneurs tels que les silicones volatiles ou non volatiles, modifiées ou non modifiées, les agents filmogènes, les céramides, les agents conservateurs, les agents opacifiants.

9. Procédé de teinture des fibres kératiniques, dans lequel, on applique sur les fibres la composition tinctoriale telle que définie à l'une quelconque des revendications 1 à 8, puis on rince lesdites fibres.

10. Procédé selon la revendication 9, tel qu'il comprend les étapes d'appliquer la composition tinctoriale selon l'une des revendications 1 à 8 sur les fibres kératiniques, puis à laisser poser pendant une période comprise entre 3 minutes et 1 heure et de préférence entre 15 minutes et 45 minutes, puis on rince lesdites fibres.

11. Utilisation d'une composition selon l'une des revendications 1 à 8 pour la teinture des fibres kératiniques.
